# EUROPEAN PATENT SPECIFICATION

(11) **EP 1 221 130 B1**
(45) Date of publication and mention of the grant of the patent: **03.05.2006**
(21) Application number: 00955224.1
(22) Date of filing: 07.08.2000
(51) Int. Cl.: G06K 9/00, A61B 5/103

(54) **FINGERPRINT SENSING APPARATUS**
GERÄT ZUR ERKENNUNG VON FINGERABDRÜCKEN
APPAREIL DE RECONNAISSANCE D'EMPREINTES DIGITALES

(30) Priority: 10.08.1999 WO PCT/SG99/00082
(43) Date of publication of application: 10.07.2002
(73) Proprietor: Nanyang Technological University, Singapore 639798 (SG)
(72) Inventor: Yau, Wei Yun Centre for Signal Processing, Singapore 639798 (SG); Jiang, Xudong Centre for Signal Processing, Singapore 639798 (SG); Ser, Wee Centre for Signal Processing, Singapore 639798 (SG)
(74) Representative: Findlay, Alice Rosemary
(86) International application number: PCT/SG2000/000114
(87) International publication number: WO 2001/011543

(56) References cited:
- EP-A- 0 566 337
- EP-A- 0 929 028
- WO-A-87/06378
- US-A- 5 644 636

## Description

The invention relates to fingerprint sensing apparatus, and especially, solid state fingerprint sensing apparatus.

Solid state fingerprint sensors are produced on a single semiconductor chip (or die) and comprise an array of sensing elements, such as capacitive sensors or electric field sensors, formed in a two dimensional array on the surface of the die.

However, this fabrication technique has the disadvantage that the die must have a surface area which is at least the same size as the fingerprint sensing area. As, the fingerprint sensing area must be large enough to accommodate the fingerprint of a user, the fingerprint sensing area must generally be at least 10mm x 10mm. If the fingerprint sensing area is much smaller than this then the area will be too small to permit the fingerprint of a user to be captured. Preferably, the fingerprint sensing area should be a larger size.

This creates a problem with die fabrication as the larger the die is the higher the probability that the die will have an error or fault. Therefore, as the die size increases, the probability of having a die in a batch with an error increases and therefore the yield of operable dies from a batch decreases. For this reason, the larger a fingerprint sensor is, the more expensive it is and the increase in cost is driven not only by the increase size, and therefore the increase in material in the die, but also the lower yield from a batch.

In addition, the requirement for larger dies also reduces the efficiency with which the silicon wafer surface area can be utilised as fewer dies can be fitted on the surface of the wafer. This also has the disadvantage of increasing the cost of solid state fingerprint sensors.

US Patent Nos. 5,907,627, 5,325,442 and 5,778,089 attempt to overcome these problems by providing a number of dies in a single packaged device.

In accordance with a first aspect of the present invention, there is provided fingerprint sensing apparatus comprising
a plurality of packaged semiconductor devices, each semiconductor device comprising a single fingerprint sensor die, each die comprising a sensing array surface, and the individually packaged semiconductor devices being arranged so that the sensing array surfaces of the dies define an apparatus sensing surface; and
a ground contact located between the sensing array surfaces of at least two adjacent semiconductor devices, the ground contact having a conducting surface raised above the surface of the sensing array surfaces.

An advantage of the invention is that, as the fingerprint sensing apparatus is formed from a number of packaged semiconductor devices, each device comprising a single fingerprint sensor die, the sensing area of the fingerprint sensing apparatus is not restricted to the surface area of one die on which a fingerprint sensor array is formed as the size of the apparatus sensing surface can be increased or decreased by using an appropriate number of fingerprint sensor dies. Therefore, fingerprint sensor dies with a relatively small sensing array surface can be used to form a fingerprint sensing apparatus with a relatively large apparatus sensing surface. In addition, the fingerprint sensing apparatus of the invention also has the advantage that the size of the area of the apparatus sensing surface can be configured relatively easily by increasing or decreasing the number of semiconductor devices.

Also, the ground contact conducting surface is raised above the surface of the sensing array surfaces which has the advantage that when a user places a finger on the apparatus sensing surface, the risk of the fingerprint touching a sensing surface before touching the grid is minimised so that any static charge on the user is discharged through the ground contact and not onto one of the sensing array surfaces. This has the advantage of minimising the possibility of a user carrying a static charge damaging the apparatus device by a static discharge onto one or more of the sensing array surfaces.

Preferably, the ground contact is located between each pair of adjacent sensing array surfaces.

In one embodiment of the invention, the ground contact is in the form of a grid.

Typically, the apparatus sensing surface is substantially planar.

In one example of the invention, the sensing array surfaces may be arranged in a one dimensional array. This arrangement is particularly useful where the length of the sensing array surface of each die is relatively large compared with the width.

Alternatively, the semiconductor devices may be arranged so that the sensing array surfaces form a two dimensional array. This arrangement is particularly useful where the width and length of the sensing array surfaces are of a similar size.

Preferably, each sensing array surface is less than 15mm x 15mm and more preferably, less than 10mm x 10mm.

In accordance with a second aspect of the present invention, there is provided a method of constructing a fingerprint image, the method comprising obtaining a number of fingerprint image portions from a finger of a user using fingerprint sensing apparatus in accordance with the first aspect of the invention, each image portion being obtained from a corresponding semiconductor device, calculating direction information at an edge of a first image portion corresponding to an edge of a first sensing array surface which is adjacent to but separated from an edge of a second sensing array surface, interpolating the direction information and pixel values at the edge of the first image portion to obtain the values of pixels between the edge of the first image portion and an edge of a second image portion corresponding to the edge of the second sensing array surface.

Preferably, the method further comprises calculating direction information at the edge of the second image portion and interpolating the direction information and the pixel values at the edges of the first and second image portions to obtain the values of pixels between the edges of the first and second image portions.

In accordance with a third aspect of the present invention, there is provided a method of constructing a fingerprint image, the method comprising obtaining a first set of fingerprint image portions from a finger of a user using a fingerprint sensing apparatus according to the first aspect of the invention, each image portion being obtained from a corresponding semiconductor device, obtaining a second set of fingerprint image portions from the fingerprint sensing apparatus with the position of the finger on the sensing apparatus offset from the position in which the first set of image portions was obtained, and comparing the first and the second sets of fingerprint image portions.

An example of fingerprint sensing apparatus in accordance with the invention will now be described with reference to the accompanying drawings, in which:-
Figure 1 shows a plan view of an apparatus sensing surface of fingerprint sensing apparatus with semiconductor devices comprising a single fingerprint sensor die arranged in a one dimensional array;
Figure 2 is a plan view of an apparatus sensing surface of a fingerprint sensing apparatus with semiconductor devices comprising a single fingerprint sensor die arranged in a two dimensional array;
Figure 3 is a perspective exploded view of a first example of fingerprint sensing apparatus having a two dimensional array;
Figure 4 is a perspective exploded view of a second example of fingerprint sensing apparatus having a two dimensional array;
Figure 5 is a cross-sectional view of the fingerprint sensing apparatus shown in Figure 3 or 4; and
Figure 6 shows a portion of two fingerprint image portions obtained using the apparatus shown in Figures 1 to 5;

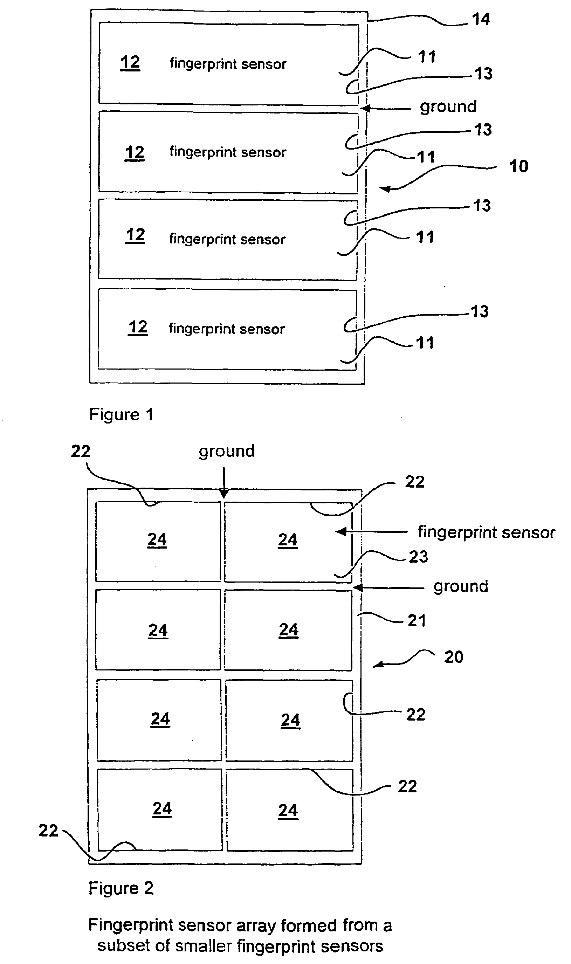

Figure 1 shows fingerprint sensing apparatus 10 which comprises four individual packaged semiconductor devices 11 each having a single fingerprint sensor die. The devices 11 are arranged in a one dimensional linear array. Each of the fingerprint sensor dies has a sensing array surface 12. Each of the fingerprint sensor dies is a conventional solid-state fingerprint sensor, such as a direct contact, fingerprint acquisition device using capacitive sensing. This type of device has an array of solid-state capacitors formed on surface 12. An example of a commercially available, solid state device 11 that could be used in the fingerprint sensing apparatus 10 is a Veridicom FPS100 produced by Veridicom, Inc. of Santa Clara, California, USA.

Each of the devices 11 has an individual controller (not shown) and all the individual controllers are multiplexed to a main central sensor controller (not shown) for the fingerprint sensing apparatus 10. The central sensor controller may also include a sensor oscillator (not shown) which can be used to ensure synchronisation of the devices 11.

As an alternative to the devices 11 comprising an array of capacitive sensors, it is possible that they could comprise an array of electric field sensors, or an array of any other suitable type of sensors.

The sensing apparatus 10 also includes a metal grid plate 14 which has four apertures 13 into which the devices 11 locate. The top surface of the grid plate 14 is raised above the sensing array surfaces 12 of the devices 11 and the plate 14 is electrically coupled to a ground contact (not shown).

Therefore, each of the sensing areas 12 together form a device sensing surface for the apparatus 10. The area of the apparatus sensing surface is the sum of the surface areas of each individual sensing array surface 12.

Figure 2 shows another fingerprint sensing apparatus 20 which includes a metallic grid plate 21 with eight apertures 22 therein. A packaged semiconductor device 23 having a sensing array surface 24 is located in each aperture 22 so that the sensor array surfaces 24 together form a device sensing surface for the apparatus 20. Therefore, the sensing surface for the apparatus 20 is eight times the size of each sensing array surface 24.

As shown in Figure 2, the sensor array surfaces 24 are arranged in a two dimensional array. As with the devices 11, described above and shown in Figure 1, the devices 23 may be any conventional solid state fingerprint sensor using a capacitive sensing array, an electric field sensing array or any other suitable type of sensing array.

As with the metallic grid plate 12, the metallic grid plate 21 is also electrically coupled to ground and the top surfaces of the grid plate 21 are raised above the surface areas 24.

In use, when a user, who is to have their fingerprint captured by the apparatus 10 or the apparatus 20, places their finger on the sensing surface, due to the presence of the metallic grid plate 14, 21, and that the surface of the grid plate 14, 21 is raised above the surface of the sensing array surfaces 12, 24, the user's finger should contact the grid plate 14, 21 before contacting the surface array surfaces 12, 24 so that any static charge on the user will be discharged through the ground plate 14, 21 rather than through one of the devices 11, 23. Therefore, this feature minimises the risk of the devices 11, 23 being damaged by a static discharge during use.

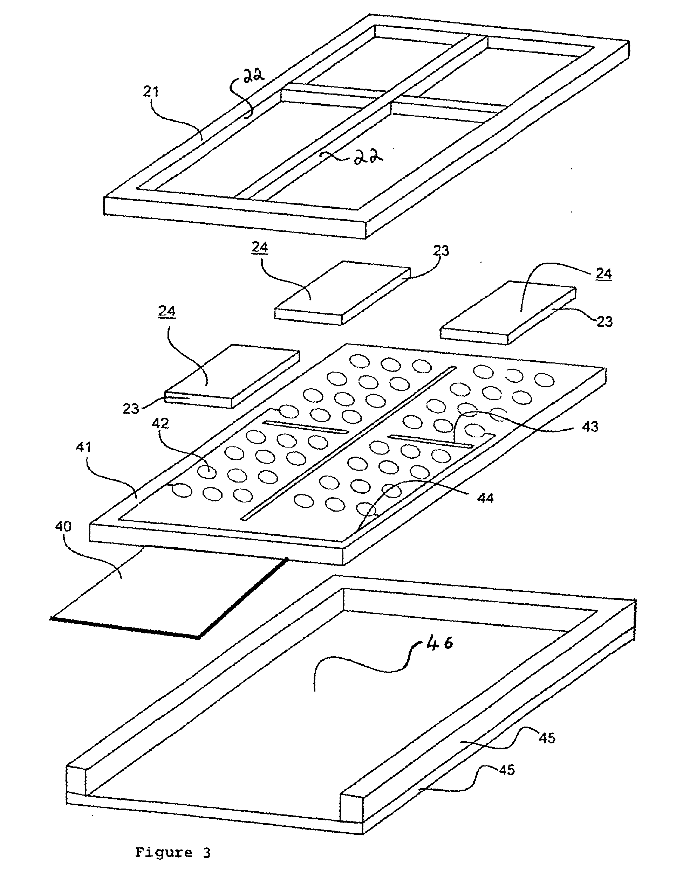

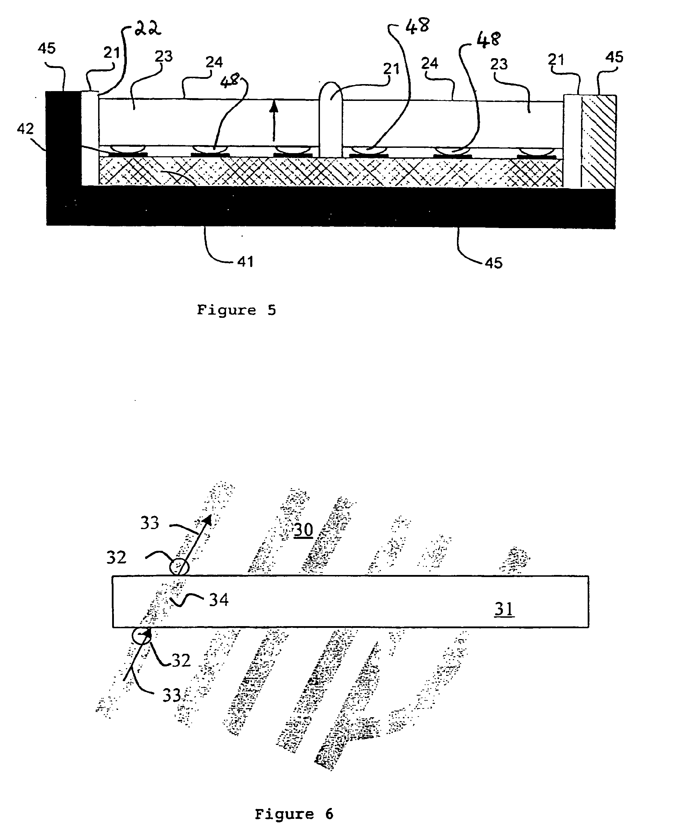

In the two examples described above, the ground plates 14, 21 are formed so that the respective devices 11, 23 fit into the respective apertures 13, 22 by being inserted from below the ground plate 14, 21. This is illustrated schematically for a two dimensional array in Figure 3 where an exploded view of a fingerprint sensing device 47 is shown. The device 47 comprises a 2 x 2 array of devices 23 which are mounted on a printed circuit board (pcb) 41 which has a number of conductive contact pads 42, and a cable 40. The cable 40 is coupled to the contact pads 42 by conductive paths 44 formed on the pcb 41. The pcb 41 is located in a holder 45 which includes a base plate 46 which supports the pcb 41. The devices 23 are mounted on the pcb 41 such that the contact pads 42 contact pads 48 on the underside of the devices 23 as shown in Figure 5. The ground plate 21 is then placed over the pcb 41 such that the devices 23 are located in the apertures 22, as shown in Figure 5.

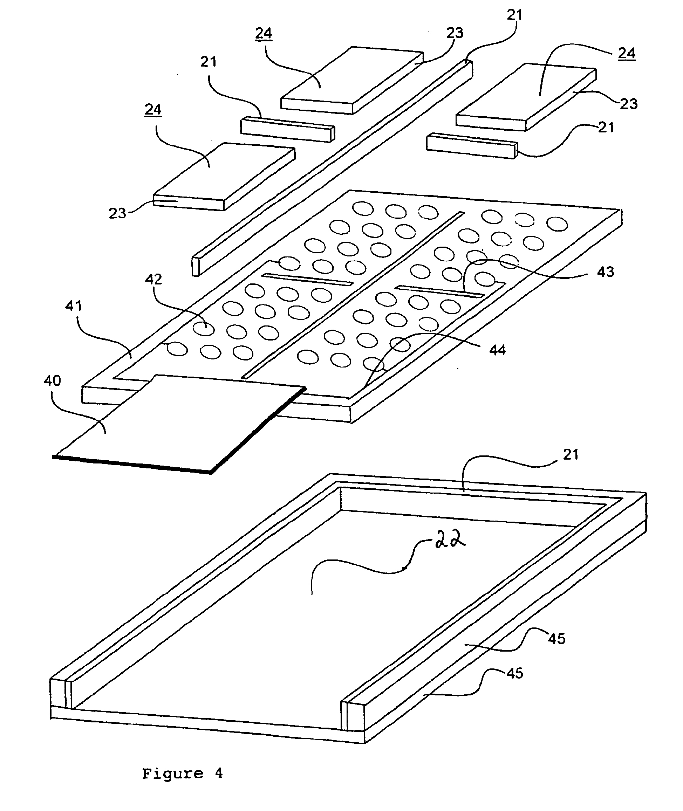

In an alternative example, it is possible that the ground contacts 21 could be formed on the pcb 41 so that when the devices 23 are mounted onto the pcb 41, the ground contacts extend up between each individual device 23 so that the upper surfaces of the ground contacts 21 are raised above the surfaces of the sensing array surfaces 24. An exploded view of this arrangement is shown in Figure 4, where parts similar to parts in Figure 3 have the same reference numeral.

In use, the various images acquired from each of the individual devices 11, 23 have to be assembled together in order to construct a complete fingerprint image. However, there are gaps 15 between adjacent devices 11 and gaps 25, 26 between adjacent devices 23 corresponding to the ground plates 14, 21. Therefore, if the images from each device 11, 23 are assembled to construct the fingerprint image, there will be discontinuities or gaps 31 (see Figure 6) in the image corresponding to the gaps 15, 25, 26. The discontinuities will cause errors in the minutiae extraction process. Therefore, it is necessary to calculate pixel values 34 for the gaps 15, 25, 26 to ensure that there are no discontinuities in the final fingerprint image. Two possible ways of filling in the missing information are:
(i) Any fingerprint image is inherently directional. Therefore, direction information 33 is useful in the construction (see Figure 6). The direction information 33 can be computed easily, using various conventional image processing methods. The size of the gap 31 and the resolution of the devices 11, 23 are known in advance from the specification of the devices 11, 23. From the specification, the number of picture elements (pixels) in the image to be allocated for the gap 31 can be calculated. However, the actual values of the pixels 34 in the gap 31 are not known. Therefore, the pixel values 34 can be estimated by using the pixel values 32 and the direction information 33 at both edges of the gap 31 by means of interpolation;
(ii) Alternatively, the fingerprint image can be acquired twice with one of the images being slightly translated from the other in both the horizontal and vertical directions. The unknown pixel values in the separation region 31 can then be estimated from the two images using conventional image processing techniques.

Advantages of the invention are that by forming fingerprint sensing apparatus 10, 20 from a number of packaged semiconductor devices 11, 23 each having a single die, it is possible to form fingerprint sensing apparatus 10, 20 with an apparatus sensing surface which is many times larger than the sensing array surface 12, 24 of each individual device 11, 23. This has the advantage that relatively small, and therefore inexpensive, devices 11, 23 can be used to form the sensing apparatus 10, 20 with a much larger sensing surface. In addition, by using a number of devices 11, 23 to form the sensing apparatus 10, 20 it is also possible to provide an electrical ground contact grid over the device sensing surface to minimise the risk of static discharge damaging the devices 11, 23 in use.

## Claims

1. Fingerprint sensing apparatus (10;20;47) comprising:
a plurality of individually packaged semiconductor devices (11;23), each semiconductor device (11;23) comprising a single fingerprint sensor die, each die comprising a sensing array surface (12;24), and the individually packaged semiconductor devices (11;23) being arranged so that the sensing array surfaces of the dies define an apparatus sensing surface; and
a ground contact located between the sensing array surfaces (12;24) of at least two adjacent semiconductor devices (11;23), the ground contact having a conducting surface raised above the surface of the sensing array surfaces.

2. Apparatus (10;20;47) according to daim 1, wherein a ground contact is located between each pair of adjacent sensing array surfaces (12;24).

3. Apparatus (10;20;47) according to claim 1 or claim 2, wherein the ground contact is in the form of a grid (14;21).

4. Apparatus (10;20;47) according to any one of the preceding claims, wherein the apparatus sensing surface is substantially planar.

5. Apparatus (10;20;47) according to any one of the preceding claims, wherein the semiconductor devices (11;23) are arranged so that the sensing array surfaces (12;24) form a one dimensional array.

6. Apparatus (10;20;47) according to any one of claims 1 to 4, wherein the semiconductor devices (11;23) are arranged so that the sensing array surfaces (12;24) form a two dimensional array.

7. Apparatus (10;20;47) according to any one of the preceding claims, wherein the sensing array surfaces (12;24) are less than 15mm x 15mm.

8. Apparatus (10;20;47) according to claim 7, wherein the sensing array surfaces (12;24) are less than 10mm x 10mm.

9. A method of constructing a fingerprint image, the method comprising obtaining a number of fingerprint image portions from a finger of a user using fingerprint sensing apparatus (10;20;47) according to any of claims 1 to 8, each image portion being obtained from a corresponding semiconductor device (11;23), calculating direction information (33) at an edge of a first image portion corresponding to an edge of a first sensing array surface (12;24) which is adjacent to but separated from an edge of a second sensing array surface (12;24), interpolating the direction information (33) and pixel values (32) at the edge of the first image portion to obtain the values of pixels (34) between the edge of the first image portion and an edge of a second image portion corresponding to the edge of the second sensing array surface (12;24).

10. A method according to claim 9, further comprising calculating direction information (33) at the edge of the second image portion and interpolating the direction information (33) and the pixel values (32) at the edges of the first and second image portions to obtain the values of pixels (34) between the edges of the first and second image portions.

11. A method of constructing a fingerprint image, the method comprising obtaining a first set of fingerprint image portions from a finger of a user using fingerprint sensing apparatus (10;20;47) according to any of claims 1 to 8, each image portion being obtained from a corresponding semiconductor device (11;23), obtaining a second set of fingerprint image portions from the fingerprint sensing apparatus (10;20;47) with the position of the finger on the sensing apparatus (10;20;47) offset from the position in which the first set of image portions was obtained, and comparing the first and the second sets of fingerprint image portions.

## Patentansprüche

1. Gerät zur Erfassung von Fingerabdrücken (10; 20; 47) mit:
einer Vielzahl von einzeln in Gehäusen untergebrachten Halbleitervorrichtungen (11; 23), wobei jede Halbleitervorrichtung (11; 23) ein einzelnes Fingerabdrucksensor-Plättchen aufweist, wobei jedes Plättchen eine Erfassungsfeldoberfäche (12; 24) aufweist, und wobei die einzeln in Gehäusen untergebrachten Halbleitervorrichtungen (11; 23) so angeordnet sind, daß die Erfassungsfeldoberfächen der Plättchen eine Erfassungsoberfläche des Gerätes bilden; und
einem zwischen den Erfassungsfeldoberfächen (12; 24) von zumindest zwei benachbarten Halbleitervorrichtungen (11; 23) angeordneten Massekontakt, wobei der Massekontakt eine sich über die Oberfläche der Erfassungsfeldoberfächen erhebende leitende Oberfläche aufweist.

2. Gerät (10; 20; 47) nach Anspruch 1, wobei zwischen jedem Paar von benachbarten Erfassungsfeldoberfächen (12; 24) ein Massekontakt angeordnet ist.

3. Gerät (10; 20; 47) nach Anspruch 1 oder 2, wobei der Massekontakt die Form eines Gitters (14; 21) hat.

4. Gerät (10; 20; 47) nach einem der vorhergehenden Ansprüche, wobei die Erfassungsoberfläche des Gerätes im wesentlichen eben ist.

5. Gerät (10; 20; 47) nach einem der vorhergehenden Ansprüche, wobei die Halbleitervorrichtungen (11; 23) so angeordnet sind, daß die Erfassungsfeldoberflächen ein eindimensionales Array bilden.

6. Gerät (10; 20; 47) nach einem der Ansprüche 1 bis 4, wobei die Halbleitervorrichtungen (11; 23) so angeordnet sind, daß die Erfassungsfeldoberflächen (12; 24) ein zweidimensionales Array bilden.

7. Gerät (10; 20; 47) nach einem der vorhergehenden Ansprüche, wobei die Erfassungsfeldoberfächen (12; 24) kleiner als 15 mm x 15 mm sind.

8. Gerät (10; 20; 47) nach Anspruch 7, wobei die Erfassungsfeldoberfächen (12; 24) kleiner als 10 mm x 10 mm sind.

9. Verfahren zur Erzeugung eines Fingerabdruckbildes, wobei das Verfahren umfaßt: Erhalten einer Anzahl von Fingerabdruckbild-Teilen von einem Finger eines Benutzers unter Verwendung eines Gerätes zur Erfassung von Fingerabdrücken (10; 20; 47) gemäß einem der Ansprüche 1 bis 8, wobei jeder Bild-Teil von einer entsprechenden Halbleitervorrichtung (11; 23) erhalten wird, Berechnen einer Richtungsinformation (33) am Rand eines ersten Bild-Teils, der dem Rand einer ersten Erfassungsfeldoberfläche (12; 24), die benachbart zu, aber getrennt von einem Rand einer zweiten Erfassungsfeldoberfläche (12; 24) ist, entspricht, Interpolieren der Richtungsinformation (33) und von Pixelwerten (32) am Rand des ersten Bild-Teils, um die Pixelwerte (34) zwischen dem Rand des ersten Bild-Teils und einem Rand eines zweiten Bild-Teils, der dem Rand der zweiten Erfassungsfeldoberfläche (12; 24) entspricht, zu erhalten.

10. Verfahren nach Anspruch 9, ferner umfassend Berechnen einer Richtungsinformation (33) am Rand des zweiten Bild-Teils und Interpolieren der Richtungsinformation (33) und der Pixelwerte (32) an den Rändern des ersten und des zweiten Bild-Teils, um die Pixelwerte (34) zwischen den Rändern des ersten und des zweiten Bild-Teils zu erhalten.

11. Verfahren zur Erzeugung eines Fingerabdruckbildes, wobei das Verfahren umfaßt: Erhalten eines ersten Satzes von Fingerabdruckbild-Teilen von einem Finger eines Benutzers unter Verwendung eines Gerätes zur Erfassung von Fingerabdrücken (10; 20; 47) gemäß einem der Ansprüche 1 bis 8, wobei jeder Bild-Teil von einer entsprechenden Halbleitervorrichtung (11; 23) erhalten wird, Erhalten eines zweiten Satzes von Fingerabdruckbild-Teilen vom Gerät zur Erfassung von Fingerabdrücken (10; 20; 47), wobei die Position des Fingers auf dem Erfassungsgerät (10; 20; 47) gegenüber der Position, in welcher der erste Satz von Bild-Teilen erhalten wurde, versetzt ist, und Vergleichen des ersten und des zweiten Satzes von Fingerabdruckbild-Teilen.

## Revendications

1. Dispositif de détection d'empreinte digitale (10; 20 ; 47) comprenant :
une pluralité de dispositifs semi-conducteurs (11 ; 23) conditionnés individuellement, chaque dispositif semi-conducteur (11 ; 23) comprenant une seule puce de détection d'empreinte digitale, chaque puce comprenant une surface de réseau de détection (12 ; 24), et les dispositifs semi-conducteurs (11 ; 23) conditionnés individuellement étant agencés de sorte que les surfaces de réseau de détection des puces définissent une surface de détection de dispositif ; et
un contact de masse situé entre les surfaces de réseau de détection (12 ; 24) d'au moins deux dispositifs semi-conducteurs (11 ; 23) adjacents, le contact de masse comportant une surface conductrice élevée au-dessus de la surface des surfaces de réseau de détection.

2. Dispositif (10 ; 20 ; 47) selon la revendication 1, dans lequel un contact de masse est situé entre chaque paire de surfaces de réseau de détection (12 ; 24) adjacentes.

3. Dispositif (10 ; 20 ; 47) selon la revendication 1 ou la revendication 2, dans lequel le contact de masse a la forme d'une grille (14 ; 21).

4. Dispositif (10 ; 20 ; 47) selon l'une quelconque des revendications précédentes, dans lequel la surface de détection de dispositif est sensiblement plane.

5. Dispositif (10 ; 20 ; 47) selon l'une quelconque des revendications précédentes, dans lequel les dispositifs semi-conducteurs (11 ; 23) sont agencés de sorte que les surfaces de réseau de détection (12 ; 24) forment un réseau unidimensionnel.

6. Dispositif (10 ; 20 ; 47) selon l'une quelconque des revendications 1 à 4, dans lequel les dispositifs semi-conducteurs (11 ; 23) sont agencés de sorte que les surfaces de réseau de détection (12 ; 24) forment un réseau bidimensionnel.

7. Dispositif (10 ; 20 ; 47) selon l'une quelconque des revendications précédentes, dans lequel les surfaces de réseau de détection (12 ; 24) sont inférieures à 15 mm x 15 mm.

8. Dispositif (10 ; 20 ; 47) selon la revendication 7, dans lequel les surfaces de réseau de détection (12; 24) sont inférieures à 10 mm x 10 mm.

9. Procédé de construction d'une image d'empreinte digitale, le procédé comprenant l'obtention d'un certain nombre de parties d'image d'empreinte digitale d'un doigt d'un utilisateur en utilisant le dispositif de détection d'empreinte digitale (10 ; 20 ; 47) selon l'une quelconque des revendications 1 à 8, chaque partie d'image étant obtenue par un dispositif semi-conducteur (11 ; 23) correspondant, le calcul d'informations de direction (33) au niveau d'un bord d'une première partie d'image correspondant à un bord d'une première surface de réseau de détection (12: 24) qui est adjacent à mais séparé d'un bord d'une deuxième surface de réseau de détection (12 ; 24), l'interpolation des informations de direction (33) et de valeurs de pixels (32) au niveau du bord de la première partie d'image afin d'obtenir les valeurs de pixels (34) entre le bord de la première partie d'image et un bord d'une deuxième partie d'image correspondant au bord de la deuxième surface de réseau de détection (12 ; 24).

10. Procédé selon la revendication 9, comprenant en outre le calcul d'informations de direction (33) au niveau du bord de la deuxième partie d'image et l'interpolation des informations de direction (33) et des valeurs de pixels (32) au niveau des bords des première et deuxième parties d'image afin d'obtenir les valeurs de pixels (34) entre les bords des première et deuxième parties d'image.

11. Procédé de construction d'une image d'empreinte digitale, le procédé comprenant l'obtention d'un premier ensemble de parties d'image d'empreinte digitale d'un doigt d'un utilisateur en utilisant un dispositif de détection d'empreinte digital (10 ; 20 ; 47) selon l'une quelconque des revendications 1 à 8, chaque partie d'image étant obtenue d'un dispositif semi-conducteur (11 ; 23) correspondant, l'obtention d'un deuxième ensemble de parties d'image d'empreinte digitale du dispositif de détection d'empreinte digitale (10 ; 20 ; 47), la position du doigt sur le dispositif de détection (10 ; 20 ; 47) étant décalée de la position à laquelle le premier ensemble de parties d'image a été obtenu, et la comparaison des premier et deuxième ensembles de parties d'image d'empreinte digitale.
